# EUROPEAN PATENT APPLICATION

(11) **EP 1 712 139 A1**
(43) Date of publication of application: **18.10.2006**
(21) Application number: 05252368.5
(22) Date of filing: 15.04.2005
(51) Int. Cl.: A23L 1/09, A23L 1/307, A23L 1/29

(54) **Sustained energy release compositions**

(71) Applicant: Cargill, Incorporated, Wayzata, Minnesota 55391 (US)
(72) Inventor: The designation of the inventor has not yet been filed
(74) Representative: Wilkinson, Stephen John

(57) **Abstract**

There is provided the use of a glucopyranosyl sugar alcohol in the preparation of a sustained energy release food, feed and/or drink composition and a method of preparing such compositions.

## Description

### Technical Field of the Invention

The present invention relates to the use of certain specified carbohydrate materials in the preparation of sustained glucose and energy release food, feed and drink compositions and to compositions containing such materials.

### Background of the Invention

Carbohydrates are formed of monomer units such as glucose, fructose and galactose. Many carbohydrates are linked to each other by α-1,4- glucosidic bonds that are easily hydrolysed during the early stages of digestion in both humans and animals (i.e. in the mouth, stomach and small intestine). Examples of such carbohydrates include the commercially available hydrolysis products of starch, such as maltose and maltodextrins. It is common knowledge that oral ingestion of such carbohydrates leads to a rapid increase in blood glucose concentration and therefore to an elevated insulin response. This is typically followed by a sharp decrease in blood glucose and, because insulin levels remain high, can result in so called "rebound hypoglycaemia". Symptoms of hypoglycaemia include nausea, weakness, hallucinations, headaches, hypothermia and fainting.

Conversely, for some individuals (such as diabetics), an increase in blood glucose (hyperglycaemia) can be maintained for a prolonged period of time.. This occurs when insulin levels are insufficient to stimulate glucose uptake by tissues (resulting in a normalisation of blood glucose) or when tissue insulin sensitivity is impaired. Such prolonged hyperglycaemia induces undesired effects on metabolism in the body often referred to as metabolic syndrome, a combination of several disease risk factors such as elevated blood pressure, impaired glucose tolerance, elevated fasting blood glucose and impaired blood lipid levels, often together with weight gain. Being exposed to these risk factors is known to lead to a significant increase in morbidity and mortality.

There is therefore a clear need to develop alternative carbohydrate compounds that can be safely ingested whilst retaining the desirable properties (such as sweetness) of more conventional carbohydrates (e.g. glucose, maltose, maltodextrins and sucrose).

A number of high intensity sweeteners have been proposed for helping to reduce the increase in blood glucose, when being substituted for absorbable glucose sources, including, for example, aspartame, saccharin, sucralose or cyclamate. Unfortunately, these sweeteners cannot be used as effective sources of glucose and energy required for maintenance of a normal cellular function of the central nervous system and red blood cells. Moreover, there are conditions in which a consistent supply of glucose is an important characteristic, e.g. in nutritional compositions used by highly active people (such as sports men and women) or by certain categories of patients that have a particularly high energy turnover (burn patients, for example).

The problem to be solved by the present invention is therefore the provision of a carbohydrate material that does not cause rebound hypoglycaemia whilst nevertheless having high sweetness and being a consistent source of glucose and energy.

Attempts have already been made in the art to address this problem. Proposed solutions include carbohydrates such as isomaltulose, certain dextrans and pullulan. All of these carbohydrates, however, have a number of drawbacks. Taking isomaltulose by way of example, although its digestion results in a slow release of glucose into the blood it only has a relatively low sweetness (42% the sweetness of sucrose), it is not very heat or acid stable (stability being a particularly desirable characteristic for compounds used in food compositions) and it has a relatively high Maillard reactivity (meaning that it can lead to undesirable browning).

The present invention therefore aims to provide an alternative to these known, slow-release carbohydrates which does not suffer from the drawbacks associated with the

### prior art.

### Statements of the Invention

In a first aspect of the present invention, there is provided the use of a glucopyranosyl sugar alcohol in the preparation of a sustained energy release food, feed and/or drink composition.

In a second aspect of the present invention, there is provided a sustained energy release food, feed and/or drink composition characterised in that it comprises a glucopyranosyl sugar alcohol.

In a third aspect of the present invention, there is provided a process for the preparation of a sustained energy release food, feed and/or drink composition characterised in that it comprises the step of adding a glucopyranosyl sugar alcohol to said composition.

### Brief Description of the Drawings

**Figure 1** shows the in-vitro digestibility of *O*-α-D-Glucopyranosyl glycerol obtained according to Example 4.
**Figure 2** shows the glycemic index of *O*-α-D-Glucopyranosyl glycerol calculated according to the results of Example 4.
**Figure 3** shows the integral blood response for *O*-α-D-Glucopyranosyl glycerol calculated according to the results of Example 4.

### Detailed Description of the Invention

As noted above, the present invention provides, in a first aspect, the use of a glucopyranosyl sugar alcohol in the preparation of a sustained energy release food, feed and/or drink composition.

### Glucopyranosyl sugar alcohols

Glucopyranosyl sugar alcohols are carbohydrate compounds composed of a glucose unit and a sugar alcohol unit. Sugar alcohols (also know as polyols) are derived from carbohydrates whose carbonyl group has been reduced to a primary or secondary hydroxyl group. They have the general formula CₙH₂ₙ₊₂Oₙ. Common sugar alcohols include mannitol, sorbitol, xylitol, lactitol, isomalt, maltitol and hydrogenated starch hydrolysates (HSH).

The glucopyranosyl sugar alcohol of the present invention will preferably be selected from a glucopyranosyl C3 sugar alcohol, a glucopyranosyl C4 sugar alcohol, a glucopyranosyl C5 sugar alcohol and mixtures of two or more thereof. More preferably, the glucopyranosyl sugar alcohol of the present invention will be selected from a *O*-α-D-glucopyranosyl triitol, a *O*-α-D-glucopyranosyl tetritol, a *O*-α-D-glucopyranosyl pentitol and mixtures of two or more thereof. Even more preferably, the glucopyranosyl sugar alcohol of the present invention will be selected from *O*-α-D-glucopyranosyl glycerol, *O*-α-D-glucopyranosyl erythritol, *O*-α-D-glucopyranosyl D-threitol, *O*-α-D-glucopyranosyl L-threitol, *O*-α-D-glucopyranosyl D-arabinitol, *O-*α-D-glucopyranosyl L-arabinitol, *O*-α-D-glucopyranosyl xylitol, *O*-α-D-glucopyranosyl D-ribitol, *O*-α-D-glucopyranosyl L-ribitol and mixtures of two or more thereof. Even more preferably, it will be selected from *O*-α-D-glucopyranosyl glycerol, *O*-α-D-glucopyranosyl erythritol and mixtures thereof.

A number of isomers of each of the glucopyranosyl sugar alcohols exists, depending on which alcohol group of the sugar alcohol unit the glucose unit is linked to. Thus, for example, *O*-α-D-glucopyranosyl glycerol can exist as *O*^{*1*}-α-D-glucopyranosyl glycerol (when the glucose unit is linked to the primary alcohol group of glycerol) or as *O*^{*2*}-α-D-glucopyranosyl glycerol (when the glucose unit is linked to the secondary alcohol group of glycerol). Preferably, the glucopyranosyl sugar alcohols of the present invention will be in their primary form (i.e. with the glucose unit is linked to the primary alcohol group of the sugar alcohol unit). Accordingly, the glucopyranosyl sugar alcohols of the present invention will most preferably be selected from *O*^{*1*}-α-D-glucopyranosyl glycerol, *O*^{*1*}-α-D-glucopyranosyl erythritol and mixtures thereof.

### Sustained energy release

Glucopyranosyl sugar alcohols were already known in the art. They were thought, however, to be indigestible or only very slightly digestible. In fact, we have now surprisingly found that they undergo a slow but total hydrolysis in the small intestine resulting, for several hours after ingestion, in a continuous low-level release of glucose into the blood. This effect is referred to herein as "sustained energy release".

Thus, the present invention further provides a sustained energy release food, feed and/or drink composition characterised in that it comprises a glucopyranosyl sugar alcohol.

### Food, Feed and Drink Compositions

Such compositions could be used, for example, to deliver carbohydrates to diabetic patients without causing clinically significant hyperglycaemia. They could also be added to the diet of overweight or elderly people suffering from reduced glucose tolerance. In the domain of sports nutrition, glucopyranosyl sugar alcohols could be used to supply athletes with a steady and constant carbohydrate supply during physical exercise. They could also be used in foods and/or supplements for growing children and in so-called "energy drinks" or "energy bars". This is, of course, a non-exhaustive list and many other potential embodiments of the present invention will be apparent to the skilled person.

### Process for the preparation of sustained energy release compositions

In a further aspect, the present invention provides a process for the preparation of a sustained energy release food, feed and/or drink composition characterised in that it comprises the step of adding a glucopyranosyl sugar alcohol to said composition.

The glucopyranosyl sugar alcohol will typically be added in an amount of at least 5% by weight. Preferably, it will be added in an amount of at least 15%, even more preferably in an amount of at least 20% by weight based on the total weight of the composition. The exact amount to be added will of course depend on various factors - such as type of application (food, feed or drink), target consumer (sports people, young children, diabetics, etc.), desired level of sweetness and caloric value of the final composition, etc. ― and will easily be calculated by the skilled person.

The glucopyranosyl sugar alcohol may be added to the food, feed and/or drink composition at any stage during its production. Advantageously, glucopyranosyl sugar alcohols have been found to have a high level of sweetness. Glucopyranosyl glycerol, for example, has approximately 55% the sweetness of sucrose (i.e. about 15% more sweetness than isomaltulose) - Biosci. Biotechnol. Biochem., 64(9), 1821-1826, 2000. Typically therefore, glucopyranosyl sugar alcohols will be added in lieu of or in combination with other carbohydrate materials or sweeteners.

### Advantages of the Invention

It has surprisingly found that, despite not causing any sudden sharp increase in blood glucose concentration, glucopyranosyl sugar alcohols can nonetheless be used as sustained energy release ingredients (they are slowly but completely digestible). Such sustained supply of energy is known to result in hormonal patterns that favour the feeling of fullness (satiation) and induce less hunger. In addition, glucopyranosyl sugar alcohols have been found to have high sweetness and low cariogenicity. Their ingestion leads to only low glycemic and insulinemic responses and therefore presents a significantly reduced risk, if any, of causing either rebound hypoglycaemia or persistent strong hyperglycaemia. A low insulinemic response is also known to promote a high rate of fatty acid mobilisation from adipose (fat) tissue resulting in a high rate of fatty acid oxidation in energy metabolism (Newsholme E.A., Leech A.R. (eds): Integration of carbohydrate and lipid metabolism. In: Biochemistry for the medical sciences. John Wiley & Sons, Chichester 1983, pp 336-35, Newsholme E.A., Start C. (eds): Adipose tissue and the regulation of fat metabolism. In: Regulation in Metabolism. John Wiley & Sons, Chichester 1973a, pp 195-246).

These features make glucopyranosyl sugar alcohols an ideal alternative to other carbohydrates or sweeteners for use in:
- food, feed and/or drink compositions aimed at supporting physical and mental performance (e.g. in fitness/sports nutrition, infant nutrition, nutrition for the elderly, in so-called "brain foods" directed, for example, at students or people whose jobs require consistently high levels of concentration and/or alertness such as pilots);
- food, feed and/or drink compositions aimed at individuals requiring slow but continuous carbohydrate energy delivery, for example in conditions that require medical nutrition and/or enteral feeding (e.g. for cachexia patients, burn patients), or for post-operative nutrition;
- food, feed and/or drink compositions aimed at inducing satiety, reducing overall energy intake and/or increasing fat metabolism (e.g. slimming products, drinks for children, etc.); and
- food, feed and/or drink compositions aimed at individuals suffering from particular metabolic disorders (such as diabetes, low glucose tolerance and metabolic syndrome (syndrome X) patients).

The present invention shall now be described by way of the following, non-limiting examples.

### Examples

### Example 1: Synthesis of O¹-α-D-Glucopyranosyl glycerol

A mixture of 4.4 1 water, 25 1 anhydrous glycerol, 9.7 kg dextrose monohydrate and 1.511 kg of Optidex L-300 (a gluco-amylase enzyme from Genencor International) was prepared and its pH adjusted to 4.5 with hydrochloric acid. The mixture was then heated to 50 °C for 48h. After filtration and passing through a strong cation exchanger followed by a weak anion exchanger, the syrup was vacuum concentrated to 35% dry substance.

The total yield of *O*-α-D-Glucopyranosyl glycerol was 26% as compared to the starting materials (using HPLC analysis).

### Example 2: Purification of O¹-α-D-Glucopyranosyl glycerol

The reaction mixture, obtained under Example 1, was subjected to a chromatographic enrichment step. A strong cationic ion exchange resin in the Na⁺ form was used. Demineralised water was used as eluant. A final product was obtained containing 93% *O*-α-D-Glucopyranosyl glycerol. The product was further analysed and the relative content of *O*^{*1*}-α-D-Glucopyranosyl glycerol and *O*^{*2*}-α-D-Glucopyranosyl glycerol was found to be 94/6 by GC-MS analysis after trimethylsilylation of the product.

### Example 3: In-vitro digestibility of O¹-α-D-Glucopyranosyl glycerol

The enriched *O*^{*1*}-α-D-Glucopyranosyl glycerol of Example 2 was used as substrate in in-vitro digestibility studies.

1% substrate solutions (w/w) of each of maltose (from Merk), isomaltulose (from ICN) and *O*^{*1*}-α-D-Glucopyranosyl glycerol (as prepared in Example 2) were prepared in a 0.05 M phosphate buffer at pH 6 and equilibrated at 37°C for 10 minutes. A suspension of 10% rat intestinal acetone powder (supplied by Sigma) was prepared in 0.05M phosphate buffer (from Merk) at pH 6 and equilibrated at 37°C for 10 minutes.

0.6 ml rat intestinal acetone powder suspension was added to 6 ml of each of the substrate solutions and mixed. The mixtures were incubated at 37°C and a 1 ml sample was taken (0 hours incubation time). Further samples were taken after 2, 4 and 6 hours of incubation. The samples were diluted with 4 ml of demineralised water and boiled for 5 minutes. After the denaturation step, each sample was filtered through a 0.45 µm filter.

The filtrate was send through a Dionex OnGuard-ATM filter. Glucose content was determined by HPLC.

The results for the in-vitro small intestinal digestion tests for maltose, isomaltose and *O*^{*1*}-α-D-Glucopyranosyl glycerol are given in table 1.

**Table 1 : Percent glucose liberated during in-vitro digestion**

| **incubation time (h)** | **0** | **2** | **4** | **6** |
|---|---|---|---|---|
| **maltose** | 0 | 85 | 91 | 96 |
| **isomaltulose** | 0 | 7 | 14 | 19 |
| ***O***^{***1***}**-α-D-Glucopyranosyl glycerol** | 0 | 7 | 12 | 21 |

It is clear form table 1 that the in-vitro digestibility characteristics of *O*^{*1*}-α-D-Glucopyranosyl glycerol are identical to those of isomaltulose. Isomaltulose is known in the art as being a sustained energy release carbohydrate.

### Example 4: In-vivo digestibility of O¹-α-D-Glucopyranosyl glycerol

Example 3 demonstrates that glucopyranosyl glycerol has a slow rate of in-vitro digestion. In order to verify this effect in-vivo, the carbohydrate was tested in a cannulated pig model. This model was chosen because pigs have a digestive physiology very close to that of humans. Moreover, it is known that humans and pigs have a very similar carbohydrate metabolism. ( Lang V, Vaugelade P, Bernard F, et al. Euglycemic hyperinsulinemic clamp to assess posthepatic glucose appearance after carbohydrate loading. 1. Validation in pigs. Am J Clin Nutr 1999;69:1174-82). Accordingly the pig can serve as an ideal model to study the digestive and metabolic effects of novel carbohydrates.

In the present study, six young pigs were operated after an overnight fast. Catheters were implanted as follows: 1) into the portal vein for portal blood sampling, 2) into the hepatic vein for hepatic blood sampling, 3) into the abdominal aorta for arterial blood sampling, 4) into the inferior caval vein for fluid infusion, and 5) into the stomach for enteral feeding of the test carbohydrates.

After surgery, the pigs were fed standard pig feed for a period of 10 days to allow for proper recovery. After the recovery period, testing was performed: after an overnight fast, glucopyranosyl glycerol was fed to the pigs, in a single dose of 1.5g/KG body weight, using an intra-gastric tube.

Glucose monohydrate was used as a control. Control tests were performed in order to obtain a valid baseline for glycemic index (GI) determination (F. Brouns, I. Bjorck, K. N. Frayn, A. L. Gibbs, V. Lang, G. Slama and T. M. S. Wolever. Glycaemic index methodology; Nutrition Research Reviews (2005), 18, 1-28)..

Blood flow rate of the portal vein was determined by a dilution technique (as described in Ten Have GAM, Bost MCF, Suyk-Wierts JCAW, van den Bogaard AEJM, Deutz NEP. Simultaneous measurement of metabolic flux in portally-drained viscera, liver, spleen, kidney and hindquarter in the conscious pig. Lab.Anim. 1996;30:347-358). Knowing the blood flow rate and the concentration of blood glucose allows for quantitative carbohydrate absorption from the gut into the blood to be calculated (Ten Have et al 1996).

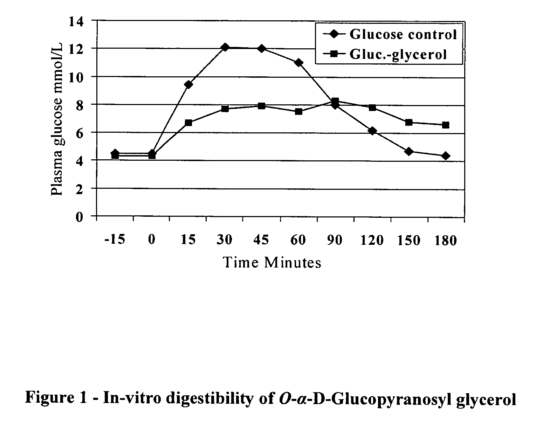

The data obtained confirmed the slow rate of digestion and of subsequent absorption of glucopyranosyl glycerol, resulting in a markedly reduced and flattened blood glucose response compared to glucose intake (see Figure 1). What is more, after glucopyranosyl glycerol ingestion, blood glucose levels remained elevated above baseline concentrations for the entire testing period. By comparison, glucose ingestion resulted in an early high blood glucose peak followed by a gradual return to baseline concentrations after only about 45 minutes.

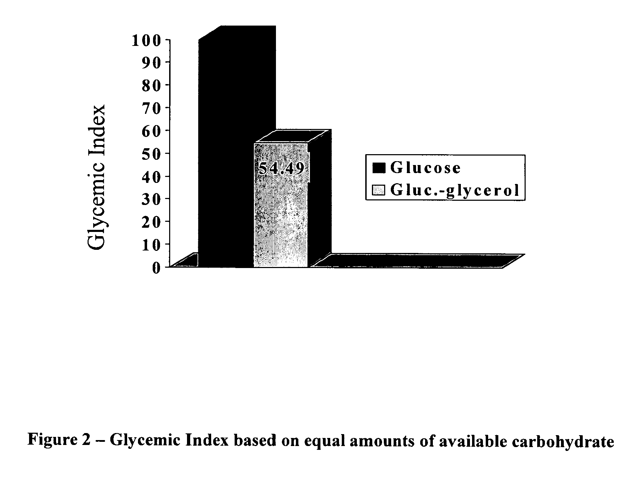

The GI of glucopyranosyl glycerol was calculated to be 54% (see Figure 2) (according to F. Brouns, I. Bjorck, K. N. Frayn, A. L. Gibbs, V. Lang, G. Slama and T. M. S. Wolever. Glycaemic index methodology; Nutrition Research Reviews (2005), 18, 1-28). Insulin levels were correspondingly low.

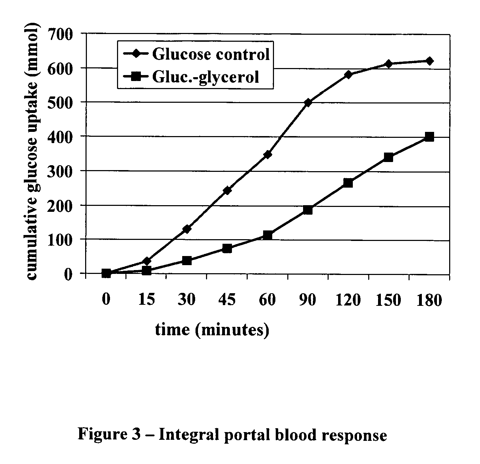

When calculating the integral blood glucose response, which is strongly dependent on both the rate of carbohydrate absorption and the related insulin response, it was shown that the absorption of glucose from glucose monohydrate ingestion leveled-off after about 90 minutes, whereas the absorption of glucose from glucopyranosyl glycerol remained at a slow but unchanged level between 90 and 180 minutes, when the test was stopped (as indicated by the linear increase over time shown in Figure 3). These results confirm that glucopyranosyl glycerol leads to a slow and sustained glucose release when digested in the small intestine.

## Claims

1. Use of a glucopyranosyl sugar alcohol in the preparation of a sustained energy release food, feed and/or drink composition.

2. Use according to claim 1 wherein the glucopyranosyl sugar alcohol is selected from a glucopyranosyl C3 sugar alcohol, a glucopyranosyl C4 sugar alcohol, a glucopyranosyl C5 sugar alcohol and mixtures of two or more thereof.

3. Use according to claim 1 or claim 2 wherein the glucopyranosyl sugar alcohol is selected from a *O*-α-D-glucopyranosyl triitol, a *O*-α-D-glucopyranosyl tetritol, a *O-*α-D-glucopyranosyl pentitol and mixtures of two or more thereof.

4. Use according to any one of claims 1 to 3 wherein the glucopyranosyl sugar alcohol is selected from *O*-α-D-glucopyranosyl glycerol, *O*-α-D-glucopyranosyl erythritol, *O*-α-D-glucopyranosyl D-threitol, *O*-α-D-glucopyranosyl L-threitol, *O*-α-D-glucopyranosyl D-arabinitol, *O*-α-D-glucopyranosyl L-arabinitol, *O*-α-D-glucopyranosyl xylitol, *O*-α-D-glucopyranosyl D-ribitol, *O*-α-D-glucopyranosyl L-ribitol and mixtures of two or more thereof.

5. Use according to any one of claims 1 to 4 wherein the glucopyranosyl sugar alcohol is selected from *O*-α-D-glucopyranosyl glycerol, *O*-α-D-glucopyranosyl erythritol and mixtures thereof.

6. Use according to claim 5 wherein the glucopyranosyl sugar alcohol is selected from *O*^{*1*}-α-D-glucopyranosyl glycerol, *O*^{*1*}-α-D-glucopyranosyl erythritol and mixtures thereof.

7. Use according to any one of claims 1 to 6 wherein the glucopyranosyl sugar alcohol is added in an amount of at least 5% by weight based on the total weight of the sustained energy release food, feed and/or drink composition.

8. A sustained energy release food, feed and/or drink composition **characterised in that** it comprises a glucopyranosyl sugar alcohol.

9. Process for the preparation of a sustained energy release food, feed and/or drink composition **characterised in that** it comprises the step of adding a glucopyranosyl sugar alcohol to said composition.
